# EUROPEAN PATENT SPECIFICATION

(11) **EP 3 353 311 B1**
(45) Date of publication and mention of the grant of the patent: **16.06.2021**
(21) Application number: 16775588.3
(22) Date of filing: 22.09.2016
(51) Int. Cl.: C12P 19/44, C12N 9/10

(54) **METHOD FOR SMALL MOLECULE GLYCOSYLATION**
VERFAHREN FÜR KLEINMOLEKULARE GLYCOSYLIERUNG
PROCÉDÉ DE GLYCOSYLATION DE PETITES MOLÉCULES

(30) Priority: 25.09.2015 EP 15186888
(43) Date of publication of application: 01.08.2018
(73) Proprietor: BASF Beauty Care Solutions France SAS, 69007 Lyon (FR)
(72) Inventor: GUDIMINCHI, Rama Krishna, 8010 Graz (AT); NIDETZKY, Bernd, 8010 Graz (AT)
(74) Representative: Cabinet Becker et Associés
(86) International application number: PCT/EP2016/072586
(87) International publication number: WO 2017/050920

(56) References cited:
- WO-A1-2014/060452
- TAEYEON KWON ET AL: "Transglucosylation of ascorbic acid to ascorbic acid 2-glucoside by a recombinant sucrose phosphorylase from Bifidobacterium longum", BIOTECHNOLOGY LETTERS, SPRINGER NETHERLANDS, DORDRECHT, vol. 29, no. 4, 10 January 2007 (2007-01-10), pages 611-615, XP019477839, ISSN: 1573-6776, DOI: 10.1007/S10529-006-9285-2
- DIRK AERTS ET AL: "Transglucosylation potential of six sucrose phosphorylases toward different classes of acceptors", CARBOHYDRATE RESEARCH, PERGAMON, GB, vol. 346, no. 13, 20 June 2011 (2011-06-20), pages 1860-1867, XP028277354, ISSN: 0008-6215, DOI: 10.1016/J.CARRES.2011.06.024 [retrieved on 2011-06-24]
- NOMURA KOJI ET AL: "Glucosylation of acetic acid by sucrose phosphorylase", BIOSCIENCE BIOTECHNOLOGY AND BIOCHEMISTRY, vol. 72, no. 1, January 2008 (2008-01), pages 82-87, XP002764765, ISSN: 0916-8451
- SUGIMOTO ET AL: "Novel transglucosylating reaction of sucrose phosphorylase to carboxylic compounds such as benzoic acid", JOURNAL OF BIOSCIENCE AND BIOENGINEERING, ELSEVIER, AMSTERDAM, NL, vol. 104, no. 1, 1 July 2007 (2007-07-01), pages 22-29, XP022196793, ISSN: 1389-1723, DOI: 10.1263/JBB.104.22
- GOEDL CHRISTIANE ET AL: "Sucrose phosphorylase: a powerful transglucosylation catalyst for synthesis of alpha-D-glucosides as industrial fine chemicals", BIOCATALYSIS AND BIOTRANSFORMATION, vol. 28, no. 1, January 2010 (2010-01), pages 10-21, XP002764766, ISSN: 1024-2422

## Description

### Field of the Invention

The present invention relates to the method for sucrose phosphorylase mediated production of 2-O-*α*-D-glucopyranosyl-L-ascorbic acid.

### Background Art

2-0-*α*-D-glucopyranosyl-L-ascorbic acid (AA-2G) is a highly stable form of all the known L-ascorbic acid derivatives. Modification of the hydroxyl group in 2-position of L-ascorbic acid (responsible for its biological activity and stability) has improved the stability of L-ascorbic acid (L-AA) significantly. Glycosylation of the hydroxyl group in 2-position of L-ascorbic acid offers several advantages over other derivatives such as phosphate and sulphate derivatives. The other isoforms of L-AA glucosides namely 3-O-*α*-D-glucopyranosyl-L-ascorbic acid (AA-3G), 5-O-*α*-D-glucopyranosyl-L-ascorbic acid (AA-5G) and 6-O-*α*-D-glucopyranosyl-L-ascorbic acid (AA-6G) have been synthesized but none of them showed stability as good as AA-2G.
a. AA-2G is an extremely inert form without showing any biological activity and reducing power and thus possesses extreme physical and chemical stability.
b. The properties of L-ascorbic acid are revealed when AA-2G is hydrolyzed into L-ascorbic acid and D-glucose by the action of *α*-glucosidase enzyme secreted by living bodies and exhibits biological activities inherent to L-ascorbic acid.
c. It has been proven that AA-2G is synthesized and metabolized *in vivo* under some specified conditions. Therefore AA-2G is recognized as the safest form of highly stabilized L-AA.
d. Because of its high solubility in water and oily substances, AA-2G is advantageously used in oral and topical formulations as a vitamin C supplement.

Glycosyl transferases (GTs) are the enzymes responsible for the synthesis of glycosides in nature whereas glycosyl hydrolases (GHs) have been evolved to degrade them. Both glycosyl transferases and glycosyl hydrolases have been successfully used for the production of a wide-variety of glycosides. These enzymes have also been used in enzymatic synthesis of AA-2G.

Rat intestinal and rice seed *α*-glucosidases, which belong to GHs, have produced AA-2G when maltose and L-AA have been used as donor and acceptor substrates respectively. But these two enzymes always inevitably produce AA-6G, AA-5G isoforms along with AA-2G. The enzymes produce only one glucoside using cheap maltose as donor substrate which is interesting for commercial applications but the enzymes are not cheaply available and the presence of isoforms rendered difficulties in isolation and purification of AA-2G. *α*-Glucosidase from *A. niger* has produced majority of AA-6G and very little AA-2G.

Cyclodextrin Glucanotransferase (CGTase), which belongs to GTs, is another well studied enzyme for the large scale production of AA-2G. EP0425066 discloses a process for producing AA-2G, which may be carried out in an industrial scale allowing a saccharide-transferring enzyme such as CGTase to act on a solution containing L-ascorbic acid and an *α*-glucosyl saccharide to form AA-2G and by-products. When CGTase is used along with cyclodextrin (the most preferred donor substrate) or starch as a donor substrate several AA-2-maltooligosaccharide by-products were inevitably formed because of the transfer of whole or part of linearized maltooligosaccharide to the L-AA. The generated several by-products having different degree of polymerization were further trimmed down to AA-2G with additional glucoamylase treatment. Use of CGTase always produced other isoforms such as AA-3G and AA-6G in relatively less amounts. The presence of these isoforms again introduced complications in downstream process especially in separation of AA-2G from other isoforms. CGTases were engineered to accept the maltose as donor substrate to avoid the formation of several by-products and additional glucoamylase treatment, but very little success was achieved and yields were not at all attractive (Han et al. references).

WO2004013344 discloses a process for producing AA-2G using *α-*isomaltosyl glucosaccharide forming enzyme (IMG) from *Arthrobacter globiformis* where AA-5G or AA-6G are not formed or formed in such a small amount that the formation of these cannot be detected (<0.1% wt/wt on dry solid basis). In this process the IMG was used as a transglycosylating enzyme along with CGTase and partial starch hydrolysate as donor substrate (to breakdown the starch into oligosaccharides). The combination of these two enzymes improved the yields and reduced the formation of AA-5G and AA-6G contents below 0.1%. However the formation of several by-products was not avoided. The by-products were further treated with glucoamylase to convert into AA-2G. Thus this system needed in total 3 enzymes to run the whole process efficiently.

The currently available enzymatic methods can produce AA-2G in large scale. The presence of several by-products and different isoforms at the end of the reaction are still bottlenecks in the whole process and are interfering in AA-2G isolation and purification. The by-products can be converted into AA-2G by glucoamylase treatment and the AA-2G can be easily separated from L-AA and glucose using strongly-acidic cation exchange resin. However AA-5G and AA-6G isoforms formed along with AA-2G are hardly separated owing to their similarity in solubilities and chromatographic properties. EP0539196 has disclosed a method of separation of AA-5G and AA-6G from AA-2G advantageously utilizing their oxidizabilities which originate from their reducing powers. This process needs an accurate control of the reaction condition to just oxidize the AA-5G and AA-6G but not allowing excess oxidation which can affect AA-2G resulting in a reduced yield of AA-2G. WO 2014/060452 discloses a method for producing AA-2G under different conditions. Taeyeon Kwon et al., Biotechnology letters, Springer Netherlands, Dordrecht, vol. 29, no. 4, 10 January 2007, pages 611-615, discloses the possibility of producing AA-2G in one step from L-AA and sucrose using recombinantly Bifidobacterium longum SPase at a pH of 7.5, which is not favorable for transfer of glycosyl to L-AA. Dirk Aerts et al., Carbohydrate Research, Pergamon, GB, vol. 346, no. 13, 20 June 2011, pages 1860-1867, does not refer to the production of AA-2G specifically even if ascorbic acid is mentioned in Table 3. The reaction identified in Table 3 with SPase of *B.ado*/*ensis,* ascorbic acid and sucrose alpha 1 phosphate is carried out at pH 7.5, which here again does not appear favorable for transfer of glycosyl to L-AA. Nomura Koji et al., Bioscience biotechnology and biochemistry, vol. 72, no. 1, January 2008, pages 82-87, discloses transglucosylation from sucrose to acetic acid by S. mutans SPase, but does not refer to AA-2G specifically. The same applies to Sugimoto et al., Journal of Bioscience and Bioengineering, Elsevier, Amsterdam, NL, vol. 104, no. 1, 1 July 2007, pages 22-29, which focus on benzoyl glucoside synthesis using the transgucosylation reaction of SPase from S. mutans.

Thus, there is still the need for an improved process for the production of AA-2G with high yields.

### Summary of invention

It is an objective of the present invention to provide an efficient one-step production process for large-scale manufacture of AA-2G.

The objective is solved by the subject of the present invention.

According to the invention there is provided a method for producing 2-0-*α*-D-glucopyranosyl-L-ascorbic acid (AA-2G) comprising the sequential steps of:
a. providing a mixture comprising a glucosyl donor and a glucosyl acceptor;
b. incubating said mixture with a sucrose phosphorylase;
c. maintaining the pH in the range of pH 4.8 to 5.5 during the incubation; and
d. optionally dosing additional glucosyl donor and/or sucrose phosphorylase during the reaction, and
e. isolating and/or purifying 2-O-*α*-D-glucopyranosyl-L-ascorbic acid,
   wherein said glucosyl donor is glucose 1-phosphate or sucrose; and
   wherein said glucosyl acceptor is ascorbic acid.

### Brief description of drawings

Fig. 1 shows the chemical structure of 2-O-*α*-D-glucopyranosyl-L-ascorbic acid (AA-2G)
Fig. 2 shows the effects of pH on AA-2G forming activity of sucrose phosphorylase enzyme of *Bifidobacterium longum.*
Fig. 3 shows the effects of temperature on AA-2G forming activity of sucrose phosphorylase enzyme of *Bifidobacterium longum.*
Fig. 4 shows the effects of donor (sucrose) and acceptor (L-AA) substrate concentrations on AA-2G forming activity of sucrose phosphorylase enzyme of *Bifidobacterium longum.*
Fig. 5 shows transglucosylations of L-ascorbic acid catalyzed by different sucrose phosphorylases.

### Description of embodiments

The present invention provides a novel and improved method for producing 2-O-*α*-D-glucopyranosyl-L-ascorbic acid comprising the sequential steps of:
a. providing a mixture comprising a glucosyl donor and a glucosyl acceptor;
b. incubating said mixture with a sucrose phosphorylase;
c. maintaining the pH in the range of pH 4.8 to 5.5 during the incubation;
d. optionally dosing additional glycosyl donor as well as sucrose phosphorylase during the reaction; and
e. isolating and/or purifying 2-O-*α*-D-glucopyranosyl-L-ascorbic acid,
wherein said glucosyl donor is glucose 1-phosphate or sucrose; and
wherein said glucosyl acceptor is ascorbic acid.
The present invention relates more specifically to the subject matters as claimed.

Thus, it was an objective to find an enzyme which can use a simple and low cost disaccharide or monosaccharide as donor substrate and perform transglycosylation only at the 2-position of L-AA.

Among the GT and GH classes, the glycoside phosphorylases (GPs) are special in several respects. GPs catalyze the phosphorolysis of *α*- and *β*-D-glycosides, mainly glucosides (Glc-OR) including disaccharides and oligo-or polysaccharides of varying degree of polymerization. Glucosyl transfer to phosphate (Pi) is favored thermodynamically *in vivo* because phosphate is usually present in large excess over *α*-D-glucose-1-phosphate (Glc-1-P). However, thermodynamic equilibrium constants (K_{eq}) of GP-catalyzed reactions fall between the K_{eq} values for the reaction of GTs (K_{eq} <<1) and GHs (K_{eq} >>1). The relatively favorable K_{eq} and the fact that phosphor-activated sugars are less expensive than nucleotide-activated ones, which are required by most GTs, make GPs interesting biocatalysts for the stereo- and regiospecific synthesis of glucosides.

Sucrose phosphorylase (SPase; EC 2.4.1.7), a glucosyl phosphorylase, catalyzes the conversion of sucrose and phosphate into D-fructose and *α*-D-glucose-1-phosphate (Glc 1-P). SPase has been isolated from a number of bacterial sources. Genes encoding SPase have been cloned from different bacteria and expressed heterologously (Kawasaki H et al., Biosci. Biotech. Biochem. (1996) 60:322-324; Kitao S and Nakano E, J. Ferment. Bioeng. (1992) 73:179-184; van den Broek LAM et al., Appl. Microbiol. Biotechnol. (2004) 65:219-227). According to the systematic sequence-based classification of glycosylhydrolases (GH) and glycosyltransferases (GT) SPase belongs to family GH13 (Clan GH-H), often referred to as the *α*-amylase family. The three-dimensional structure of SPase from *Bifidobacterium adolescentis* has been solved recently, revealing an (beta/alpha)8 barrel fold and a catalytic site in which two carboxylate groups probably fulfill the role of a nucleophile (Asp192) and a general acid/base (Glu232).

"Sucrose phosphorylase" as used herein refers not only to enzymes of the EC 2.4.1.7 class but also to molecules or functional equivalents thereof which exhibit the same properties in relation to its substrates and products. "Functional equivalents" or analogs of the enzyme are, within the scope of the present invention, various polypeptides thereof, which moreover possess the desired biological function or activity, e.g. enzyme activity.

One embodiment of the invention relates to a method for producing AA-2G, wherein the SPase is of metagenomic, microbial or bacterial origin. As used herein the term "metagenomics" refers to genetic material directly recovered from environmental samples.

A further embodiment of the invention relates to a method for producing AA-2G, wherein the SPase is of microbial or bacterial origin, preferably of bacterial origin.

Homodimeric enzyme refers to an enzyme complex formed by two identical molecules. Some of the SPase are capable of forming homodimers. Examples of homodimeric SPases are amongst others derived from *Bifidobacterium adolescentis, Streptococcus mutans,* or *Bifidobacterium longum.* Surprisingly, homodimeric SPases exhibit high site-selectivity in glycosylation of L-AA. Thus, in one embodiment of the invention the sucrose phosphorylase is a homodimeric sucrose phosphorylase.
Due to the high site selectivity of the homodimeric SPases, substantially no AA-6G by-product is formed. Thus, in one embodiment of the invention a method for producing AA-2G is provided, wherein substantially no by-product is formed. According to a further embodiment of the invention, substantially no AA-3G, AA-5G or AA-6G is formed during the incubation step.

As used herein, the term "by-product" refers to any undesired product, specifically refers to AA-3G, AA-5G or AA-6G. The term "substantially free of by-product" means that the content of the by-product is less than 25%, less than 20 %, 15%, 10%, 5%, or less than 1% by weight.

One embodiment of the invention relates to the sucrose phosphorylase showing high stability at a pH below 7, preferably at a pH below 6, more preferred at a pH below 5 and most preferred at a pH below 4.

In particular embodiments, the sucrose phosphorylase of the invention has a residual sucrose phosphorylase activity after incubation for 48 hours at 60° C and at pH selected from 2.0, 3.0, 4.0, 5.0, 6.0, and 7.0, of at least 50%, preferably at least 55%, 60%, 65%, 70%, 75%, 80%, 82%, 84%, 86%, 90%, 92%, or at least 94%, relative to the sucrose phosphorylase activity at 0 hours (before start of the incubation). In preferred embodiments, the incubation pH is 2.0, 2.5, 3.0, 3.5, 4.0, 4.5, 5.0, 5.5, 6.0, 6.5 or 7.0. In even more preferred embodiments, the incubation pH is 5.2, and the residual activity is at least 50%, 55%, 60%, 65%, 70%, 75%, 80%, or at least 82%.

SPase activity was determined at 30° C using a continuous coupled enzymatic assay, in which production of Glc 1-P from sucrose and inorganic phosphate is coupled to the reduction of NAD+ in the presence of phosphoglucomutase (PGM) and glucose 6-phosphate dehydrogenase (G6P-DH) as described in Example 2.

One embodiment of the invention relates to the sucrose phosphorylase recombinantly produced using genetic material directly obtained from environmental samples.

The advantage of using microbial sucrose phosphorylases is the simple production and isolation and stability of these enzymes. They can be obtained from microorganisms naturally or recombinantly expressing SPase.

One embodiment of the invention relates to sucrose phosphorylase obtained from *Agrobacterium vitis, Bifidobacterium adolescentis, Bifidobacterium longum, Escherichia coli,* preferably *Escherichia coli 06, Lactobacillus acidophilus, Lactobacillus delbrueckii subsp. lactis, Leuconostoc mesenteroides, Listeria monocytogenes, Pseudomonas putrefaciens, Pseudomonas saccharophila, Rhodopirellula baltica, Shewanella baltica, Shewanella frigidimarina, Solibacter usitatus, Streptococcus mutans* or *Synechococcus sp.*

One embodiment of the invention relates to recombinantly produced sucrose phosphorylase, preferably as a full-length protein or a catalytically active fragment thereof or a fusion protein. Methods for the recombinant production of enzymes are known to the person skilled in the art (e.g. Sambrook J. et al. Molecular cloning: a laboratory manual. ISBN 0-87969-309-6).

As used herein, "full-length protein" refers to sucrose phosphorylase protein encoded by a gene derived from an organism as, for instance, listed above. Said naturally occurring gene, in particular the sucrose phosphorylase encoding region of said gene, is directly employed for the recombinant production of SPase.

"A catalytically active fragment" of a sucrose phosphorylase refers to protein fragments of sucrose phosphorylase which have the same or substantially the same activity and substrate specificity as native SPase. The length of the fragments is not crucial provided that the fragments will have the same or similar substrate specificity and catalyze the formation of the same products as native sucrose phosphorylase.

The term "fusion protein" refers to sucrose phosphorylase or catalytically active fragments thereof recombinantly fused to at least one further protein, polypeptide or peptide. Said at least one further protein, polypeptide or peptide may be of any kind (e.g. enzyme).

It is noted that within the scope of the invention also functional variants (i.e. mutations including deletions, substitutions and insertions) of sucrose phosphorylase are encompassed, provided that these functional variants have the same or substantially the same activity as the native sucrose phosphorylase.

However, it is of course also possible to use sucrose phosphorylase directly obtained from the organism which naturally produces said sucrose phosphorylase.

The SPase may be employed in the incubation step as either a cell-free enzyme, which may but need not be partially purified, a whole-cell system pretreated physically or chemically for improved permeability of the cell membrane (permeabilization) and mechanical stability, encapsulated catalyst in which said free enzyme or whole-cell system are entrapped, preferably in gel-like structures, or immobilized on a carrier. Advantageously the SPase is immobilized on a carrier which preferably is a solid support. The carrier is preferably a chromatography resin, preferably selected from the group consisting of anion exchange chromatography resin, cation exchange chromatography resin, affinity chromatography resin (e.g. comprising immobilized SPase specific antibodies) and hydrophobic interaction chromatography resin.

The SPase of the present invention may be immobilized (temporarily or covalently) on any carrier, preferably particles (e.g. beads), in particular chromatography resin, provided that the enzymatic activity of the enzyme is not affected in a way to change its substrate specificity or to reduce its activity to low conversion rates.

In a further embodiment of the invention the sucrose phosphorylase is used in the form of whole-cell, cell free extract, crude, purified or immobilized form.

The reaction of SPase proceeds with net retention of the anomeric configuration and occurs through a double displacement mechanism involving two configurationally inverting steps: cleavage of the carbon-oxygen bond of the glucosyl donor and formation of a covalent *β*-glucosyl-enzyme (*β*-Glc-E) intermediate; and reaction of the intermediate with phosphate to yield Glc 1-P. In a side reaction, the *β*-Glc-E intermediate may be intercepted by water, leading to hydrolysis. Hydrolytic conversion of sucrose is irreversible but proceeds nearly two orders of magnitude slower than the phosphorolytic reaction. SPase also catalyzes transglucosylation reactions which occur in competition with hydrolysis and whereby the *β*-Glc-E intermediate is attacked by external nucleophiles and new *α*-D-glucosides are produced.

Biochemical studies have shown that SPase is strictly specific for transferring a glucosyl moiety and does not tolerate structural modifications on the glucopyranosyl ring including epimerization and deoxygenation.

The glucosyl donor to be employed in the method of the present invention is one which serves as substrate for the transglycosylation reaction catalyzed by the SPase and is sucrose or Glc 1-P. Among these two known glycosyl donors, sucrose is a cheap and highly stable high-energy glucosyl donor and weakly hydrolyzed by SPase.

Preferably the glucosyl donor is sucrose.

By contrast, the specificity of SPase for glucosyl acceptors is comparably relaxed.

The invention relates to a method for producing AA-2G, wherein the glucosyl acceptor is ascorbic acid.

Stereochemically pure glucosylglycerol was obtained in high yields of ≥90% donor substrate converted and the concentration was close to 1 M (250 g/L) when the glycerol acceptor was typically ≤ 2.5-fold molar excess over sucrose (Goedl et al., Angew Chem Int Ed Engl. 2008;47(52):10086-9). The acceptor promiscuity of different SPase enzymes determined by different research groups has been excellently reviewed by Goedl et al. (Biocatal Biotrans. 2010; 28(1):10-21). The optimum pH for glycosyl transfer reactions using SPase was from 6.5 to 7.5. Interestingly the optimum pH of 6.5 of sucrose phosphorylase (from *Streptococcus mutans*) for glucosylation of phosphate and hydroquinone was shifted to below 5.0 pH when acetic acid was used as glucosyl acceptor which indicates the requirement of protonated form of interacting group of acceptor for effective transfer of glucosyl unit.

Kwon et al. (Biotechnol Lett. 2007 Apr;29(4):611-615) disclosed the possibility of producing AA-2G in one step from L-AA and sucrose using recombinantly produced *Bifidobacterium longum* sucrose phosphorylase. The concentrations of L-AA and sucrose used were 0.5 % (w/v) and 30 % (w/v) respectively and never attempted at large scale. The results didn't disclose the concentrations of AA-2G achieved. Aerts et al. (Carbohydr Res. 2011 Sep 27;346(13):1860-7) compared transglucosylation activity of six different SP enzymes on 80 putative acceptors. Although the transglucosylated products could be clearly observed using sucrose phosphorylase from *Bifidobacterium adolescentis* when 65 mM of L-AA and 50 mM of sucrose were used as acceptor and donor substrates respectively, the transglucosylation rate was about 100 times lower than the rate of hydrolysis. Thus L-AA was described as a weak acceptor of sucrose phosphorylase. The protein engineering approaches were employed to introduce protein ligand interactions in sucrose phosphorylase for the transglucosylation of L-ascorbic acid but were not successful. These studies have not proved the practical application of sucrose phosphorylase in production of AA-2G. In the above mentioned studies the reactions were performed at 37° C and pH 7.0 to 7.5 which is not at all favorable for transfer of glucosyl unit to L-AA. No attempts were made to check the possibility at acidic pH.

In objective of the present invention was the glycosylation of L-AA to produce AA-2G in a single step without producing several byproducts or isoforms of AA-2G using SPase. Sucrose phosphorylase enzyme from *Bifidobacterium longum, Bifidobacterium adolescentis, Leuconostoc mesenteroides, Lactobacillus acidophilus* and *Streptococcus mutans* were used to check the glycosylation efficiency and specificity of L-AA. All the SPases were successful in glycosylating L-AA at acidic pH. *Streptococcus mutans, B. adolescentis* SPase and *B. longum* SPases had produced only AA-2G. The reagent concentrations and reaction conditions were systematically optimized targeting for AA-2G production in high yield. As a result, the inventor of the present invention unexpectedly found that AA-2G was formed in a remarkable amount when the reaction was conducted at a pH about 5, between 40-50° C and with 1.5 fold excess L-AA. At the end of the reaction AA-2G, L-AA, sucrose, fructose and very little glucose were observed in the reaction mixture along with an unidentified impurity at less than 3 % w/w. No other byproducts or isoforms were formed or formed in such amount that they could have been detected.

A further embodiment of the invention relates to the method of producing AA-2G, wherein the incubation step is carried out at a pH range of 4.8 to 5.5. Surprisingly, the site selectivity is strongly improved by working at a pH in the range of about 4.8 to 5.5, or at a pH of about 5.2. The reaction at an unsuitable pH would lead to poor site-selectivity. In a further embodiment of the invention the incubation step is carried out at a pH of about 5.2.

The pH of the reaction medium can be adjusted using simple organic and inorganic materials or using different types of buffer mixtures. The pH can be adjusted before initiating the reaction by enzyme addition. The pH can be also adjusted during the reaction to maintain the desired pH. The pH can be adjusted manually or in automatic way.

A further embodiment of the invention relates to the method of producing AA-2G, wherein the incubation step is carried out at a temperature range of about 30 to 70° C, or in a range of about 35 to 65° C, or in a range of about 40 to 60°

C, or in a range of about 40 to 50° C. In a further embodiment of the incubation step is carried out at a temperature of about 40° C.

A further embodiment of the invention relates to the method of producing AA-2G, wherein the incubation step is performed for at least 24 h, preferably for at least 48 h, more preferred for at least 72 h.

A further embodiment of the invention relates to the method of producing AA-2G, wherein the glucosyl acceptor is used in 0.3 to 3 fold molar excess to glucosyl donor. In a further embodiment of the invention the glucosyl acceptor is used in 0.5 to 2.5 fold molar excess to glucosyl donor, or in 1.0 to 2 fold molar excess. In a further embodiment of the invention the glucosyl acceptor is used in 1.5 fold molar excess to glucosyl donor. In a further embodiment of the invention the glucosyl acceptor is ascorbic acid and the glucosyl donor is sucrose.

A further embodiment of the invention relates to a method of producing AA-2G, wherein the amount of sucrose phosphorylase in the reaction mixture is in the range of 1 U/mL to 10,000 U/mL, or in the range of 5 U/mL to 100 U/mL, or in the range of 10 U/mL to 50 U/mL, or in the range of 20 U/mL to 40 U/mL. In a further embodiment of the invention the sucrose phosphorylase is used in an amount of about 30 U/mL.

A further embodiment of the invention relates to a method of producing AA-2G, wherein additional sucrose phosphorylase and sucrose are added to the reaction mixture during incubation to maintain sucrose phosphorylase in the range of 1 U/mL to 10,000 U/mL, or in the range of 5 U/mL to 100 U/mL, or in the range of 10 U/mL to 50 U/mL, or in the range of 20 U/mL to 40 U/mL, or at about 30 U/mL and sucrose in the range of 100 to 2,000 mM, or in the range of 250 mM to 1,000 mM or about 800 mM.

A further embodiment of the invention relates to a method of producing AA-2G, wherein sucrose phosphorylase and sucrose are added simultaneously or at different time points to the reaction mixture to maintain the required amounts as described above.

The present invention is further illustrated by the following figures and Examples without being restricted thereto.

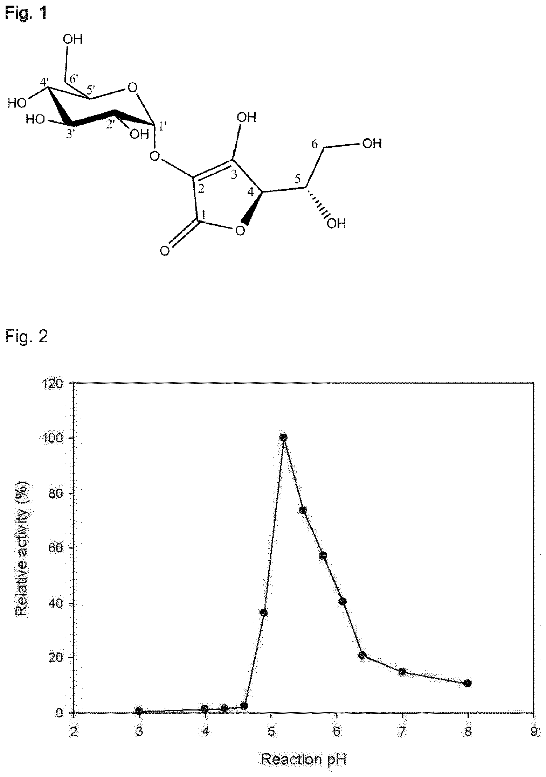

Fig. 1 shows the chemical structure of 2-O-*α*-D-glucopyranosyl-L-ascorbic acid (AA-2G)

Fig. 2 shows the effects of pH on AA-2G forming activity of sucrose phosphorylase enzyme of *Bifidobacterium longum.* An unusual pH dependence of the synthesis of AA-2G was observed. At pH 7.0 - 7.5 hardly any AA-2G was formed. Activity increased sharply on lowering the pH, reaching a distinct maximum at pH 5.2. Further decrease in the pH resulted in strong activity loss.

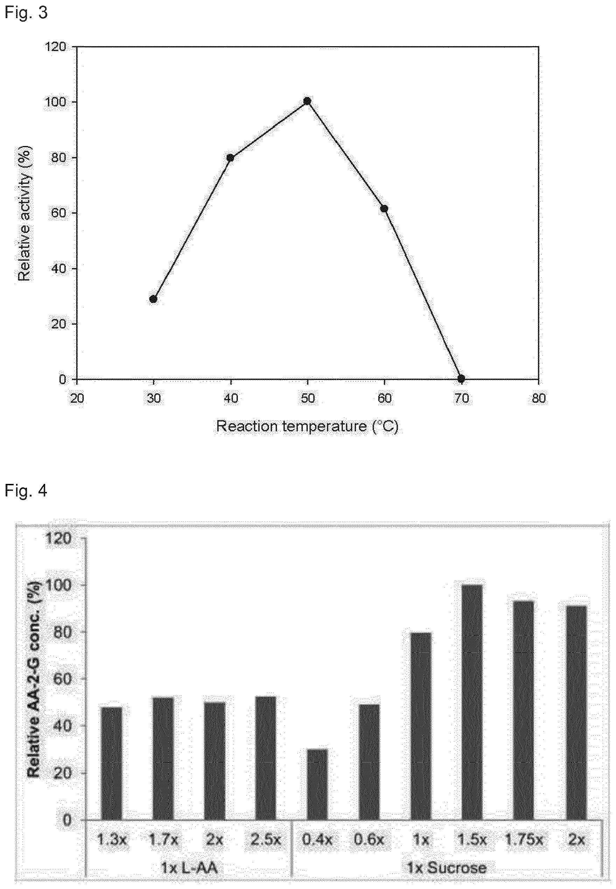

Fig. 3 shows the effects of temperature on AA-2G forming activity of sucrose phosphorylase enzyme of *Bifidobacterium longum.* The optimum temperature of AA-2G synthesis was 50° C. However, to minimize degradation of L-ascorbic acid in the process, a lower temperature of 40° C is preferable.

Fig. 4 shows the effects of donor (sucrose) and acceptor (L-AA) substrate concentrations on AA-2G forming activity of sucrose phosphorylase enzyme of *Bifidobacterium longum.* The AA-2G synthesis increased significantly with increasing L-AA concentration. The concentration and yield of AA-2G were maximized when 1.5-fold excess of L-AA was added to the reaction.

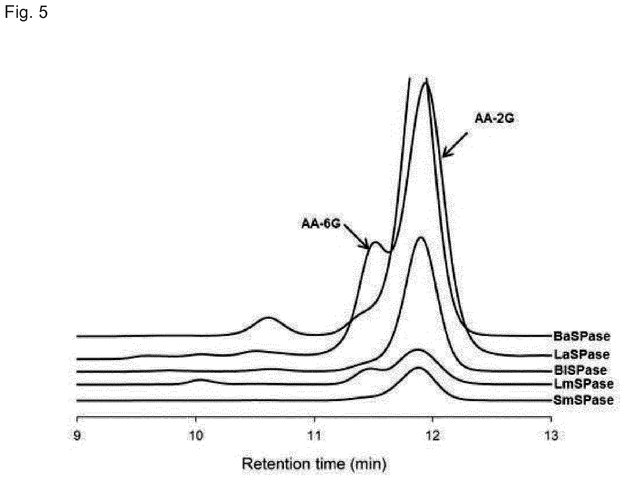

Fig. 5 shows transglucosylations of L-ascorbic acid catalyzed by different sucrose phosphorylases. The selected sucrose phosphorylases representing the sequence and structural diversity within the protein family exhibited clear differences in site-selectivity. The results are compared in Table 1.

### Examples

The Examples which follow are set forth to aid in the understanding of the invention but are not intended to, and should not be construed to limit the scope of the invention in any way. The Examples do not include detailed descriptions of conventional methods, e.g., cloning, transfection, and basic aspects of methods for expressing proteins in microbial host cells. Such methods are well known to those of ordinary skill in the art.

### Example 1 - Production of sucrose phosphorylase enzyme

The *E. coli*BL21 host strain carrying pC21E vector with an insert of SPase gene under his-tag was used for the production of SPase. The gene was cloned under tac1 promoter system and was induced with IPTG. Little amount of culture from the glycerol stock was scratched with micropipette tip and inoculated into 50 mL of LB medium containing ampicillin (120 µg/mL). The flask was incubated at 30°

C overnight on shaker at 120 rpm for about 12 h. The overnight culture was inoculated into 200 mL LB medium containing ampicillin (120 µg/mL) to generate the OD₆₀₀ of 0.01. The flask was incubated at 37° C, 120 rpm for several hours until the OD₆₀₀ reach to 0.8 to 1.0. IPTG was added to the flask to make the final concentration of 1 mM. The flask was incubated on shaker at 25° C, 120 rpm for nearly 20 h. The cells were harvested by centrifugation at 5,000 rpm for 15 min. The supernatant was decanted and the pellet was washed with 100 mM citrate buffer pH 5.2 (5 mL of buffer for each 1 g of wet cell weight was used). The 1 g of wet cells was resuspended in 5 mL of lysis buffer (100 mM citrate buffer pH 5.2 containing 50 mM NaCl + 1 mM EDTA + 0.5 mM DTT). The suspension was passed 2 times through French press. The resulting cell lysate was centrifuged at 8,000xg to separate the soluble fraction from unbroken cells and cell debris. The SPase enzyme activity in the supernatant was quantified, aliquoted and stored at - 20° C for future use.

### Example 2: Enzyme assay

SPase activity was determined at 30° C using a continuous coupled enzymatic assay, in which production of Glc 1-P from sucrose and inorganic phosphate is coupled to the reduction of NAD+ in the presence of phosphoglucomutase (PGM) and glucose 6-phosphate dehydrogenase (G6P-DH). The standard assay was performed essentially as described elsewhere in 50 mM potassium phosphate buffer, pH 7.0, containing 10 mM EDTA, 10 mM MgCl₂ and 10 µM *α*-D glucose 1,6-bisphosphate. The reaction mixture contained 250 mM sucrose, 2.5 mM NAD⁺, 3 U/mL PGM, 3.4 U/mL NAD+-dependent G6P-DH and the enzyme solution in appropriate dilution. The formation of NADH with time was monitored spectrophotometrically at 340 nm. 1 Unit of SPase activity corresponds to the amount of enzyme that caused the reduction of 1 micromol of NAD⁺ per minute under the conditions described above. Protein concentrations were determined using the BioRad dye-binding method with bovine serum albumin as standard.

### Example 3: Synthesis of 2-O-α-D-glucopyranosyl-L-ascorbic acid

The reaction mixture, containing 1,200 mM of L-AA and 800 mM of sucrose or glucose-1-phosphate and 30 U/mL of SPase in water, was incubated at 50° C and 300 rpm for 48 to 72 h. The reaction was performed preferably at pH 5.2 in air tight container under dark conditions. Product analysis was done using HPLC employing a BioRad HPX-87H column and the elution profile of the peaks were monitored with UV and RI detector. The column was maintained at 25° C and 20 mM sulfuric acid was used as eluent at a flow rate of 0.4 mL/min. Under these conditions > 30 % of L-AA was glucosylated. The NMR analysis of isolated and purified product confirmed the *α*-1-2 glycosidic linkage of AA-2G.

### Example 4: Improving the yields of 2-O-α-D-glucopyranosyl-L-ascorbic acid

The reaction mixture, containing 1,200 mM of L-AA and 800 mM of sucrose or glucose-1-phosphate and 30 U/mL of SPase in water, was incubated at 50° C and 300 rpm. During the reaction the depletion of sucrose and loss in activity of SPase was monitored. Additional sucrose and SPase were dosed after 24 h, 48 h and 72 h to restore sucrose concentration and SPase activity to their initial amounts, that is, 800 mM and 30 U/mL respectively. This way the yields were increased about 1.5x.

### Example 5: Evaluation of additional sucrose phosphorylases

4 additional sucrose phosphorylases, representing the sequence diversity within the protein family, from *Bifidobacterium adolescentis, Streptococcus mutans, Lactobacillus acidopholus, Leuconostoc mesenteroides* have been evaluated. Homodimeric sucrose phosphorylases exhibited high site-selectivity in glycosylation of L-ascorbic acid at 2-OH resulting in AA-2G formation compared to monomeric type where a mixture of AA-2G and AA-6G were formed. Monomeric sucrose phosphorylases released AA-6G in substantial proportion of total product, whereas, with homodimeric proteins no AA-6G formed above the detection limit. However, the AA-2G synthesis at pH 5.2, which is essentially lacking at pH 7.5, was an essential characteristic of all sucrose phosphorylases tested.

**Table 1**

| Sucrose Phosphorylases | AA-2G [%] | AA-6G [%] |
|---|---|---|
| *Streptococcus mutans (SmSPase)* | 100 | 0 |
| *Lactobacillus acidopholus (LaSPase)* | 77 | 23 |
| *Bifidobacterium longum (BISPase)* | 100 | 0 |
| *Leuconostoc mesenteroides (LmSPase)* | 77 | 23 |
| *Bifidobacterium adolescentis (BaSPase)* | 100 | 0 |

## Claims

1. A method for producing 2-O-α-D-glucopyranosyl-L-ascorbic acid comprising the sequential steps of:
a. providing a mixture comprising a glucosyl donor and a glucosyl acceptor;
b. incubating said mixture with a sucrose phosphorylase;
c. maintaining the pH in the range of pH 4.8 to 5.5 during the incubation;
d. optionally dosing additional glycosyl donor and/or sucrose phosphorylase during the reaction; and
e. isolating and/or purifying 2-O-α-D-glucopyranosyl-L-ascorbic acid;
wherein said glucosyl donor is glucose 1-phosphate or sucrose; and wherein said glucosyl acceptor is ascorbic acid.

2. The method according to claim 1, wherein the sucrose phosphorylase is of metagenomic, microbial, preferably of bacterial origin.

3. The method according to claim 1 or 2, wherein the sucrose phosphorylase is homodimeric.

4. The method according to any one of claims 1 to 3, wherein the bacterial sucrose phosphorylase is obtained from *Agrobacterium vitis, Bifidobacterium adolescentis, Bifidobacterium longum, Escherichia coli, Escherichia coli 06, Lactobacillus acidophilus, Lactobacillus delbrueckii subsp. lactis, Leuconostoc mesenteroides, Listeria monocytogenes, Pseudomonas putrefaciens, Pseudomonas saccharophila, Rhodopirellula baltica, Shewanella baltica, Shewanella frigidimarina, Solibacter usitatus, Streptococcus mutans* and/or *Synechococcus sp.*

5. The method according to claim 4, wherein the bacterial sucrose phosphorylase is obtained from *Bifidobacterium adolescentis.*

6. The method according to claim 4, wherein the bacterial sucrose phosphorylase is obtained from *Bifidobacterium longum.*

7. The method according to claim 4, wherein the bacterial sucrose phosphorylase is obtained from *Streptococcus mutans.*

8. The method according to any one of claims 1 to 7, wherein the sucrose phosphorylase is a native protein or a mutant variant.

9. The method according to any one of claims 1 to 8, wherein the sucrose phosphorylase is recombinantly produced as full-length protein or catalytically active fragment thereof, or fusion protein.

10. The method according to any one of claims 1 to 9, wherein the sucrose phosphorylase is used in the form of whole-cell, cell free extract, crude, purified or immobilized form.

11. The method according to any one of claims 1 to 10, wherein the incubation is performed at a pH of 5.2.

12. The method according to any one of claims 1 to 11, wherein the incubation step is performed for at least 24 h, preferably for at least 48 h, more preferred for at least 72 h.

13. The method according to any one of claims 1 to 12, wherein the incubation is performed at a temperature range of about 30 to 70 °C, preferably of about 40 to 60 °C, more preferred of about 40 to 50 °C.

14. The method according to any one of claims 1 to 13, wherein the ascorbic acid is used in 0.3 to 3 fold molar excess to sucrose.

15. The method according to any one of claims 1 to 14, wherein the amount of sucrose phosphorylase in the reaction mixture is in the range of 1 U/mL to 10,000 U/mL, or in the range of 5 U/mL to 100 U/mL, or in the range of 10 U/ml to 50 U/mL, or in the range of 20 U/mL to 40 U/mL, or 30 U/mL.

16. Method according to any one of claims 1 to 15, wherein additional sucrose phosphorylase and sucrose are added to the incubation mixture during incubation to maintain sucrose phosphorylase in the range of 1 U/ml to 10,000 U/ml, or in the range of 5 U/ml to 100 U/ml, or in the range of 10 U/ml to 50 U/ml, or in the range of 20 U/ml to 40 U/ml, or at 30 U/ml and sucrose in the range of 100 to 2,000 mM, or in the range of 250 mM to 1,000 mM or 800 mM.

17. The method according to claim 16, wherein sucrose phosphorylase and sucrose are added simultaneously or at different time points.

## Patentansprüche

1. Verfahren zur Herstellung von 2-O-α-D-Glucopyranosyl-L-ascorbinsäure, umfassend die aufeinander folgenden Schritte:
a. Bereitstellen einer Mischung, die einen Glucosyldonor und einen Glucosylakzeptor umfasst;
b. Inkubieren der Mischung mit einer Saccharosephosphorylase;
c. Halten des pH-Werts im Bereich von 4,8 bis 5,5 während der Inkubation;
d. gegebenenfalls Dosierung von zusätzlichem Glycosyldonor und / oder Saccharosephosphorylase während der Reaktion; und
e. Isolieren und/oder Reinigen von 2-O-α-D-Glucopyranosyl-L-Ascorbinsäure;
wobei dieser Glucosyldonor Glucose-1-phosphat oder Saccharose ist; und wobei dieser Glucosylakzeptor Ascorbinsäure ist.

2. Verfahren nach Anspruch 1, wobei die Saccharosephosphorylase metagenomischen, mikrobiellen, vorzugsweise bakteriellen Ursprungs ist.

3. Verfahren nach Anspruch 1 oder 2, wobei die Saccharosephosphorylase homodimer ist.

4. Verfahren nach einem der Ansprüche 1 bis 3, wobei die bakterielle Saccharosephosphorylase aus *Agrobacterium vitis, Bifidobacterium adolescentis, Bifidobacterium longum, Escherichia coli, Escherichia coli 06, Lactobacillus acidophilus, Lactobacillus delbrueckii subsp. lactis, Leuconostoc mesenteroides, Listeria monocytogenes, Pseudomonas putrefaciens, Pseudomonas saccharophila, Rhodopirellula baltica, Shewanella baltica, Shewanella frigidimarina, Solibacter usitatus, Streptococcus mutans* und/oder *Synechococcus sp.* erhalten wird.

5. Verfahren nach Anspruch 4, wobei die bakterielle Saccharosephosphorylase aus *Bifidobacterium adolescentis* erhalten wird.

6. Verfahren nach Anspruch 4, wobei die bakterielle Saccharosephosphorylase aus *Bifidobacterium longum* erhalten wird.

7. Verfahren nach Anspruch 4, wobei die bakterielle Saccharosephosphorylase aus *Streptococcus mutans* erhalten wird.

8. Verfahren nach einem der Ansprüche 1 bis 7, wobei die Saccharosephosphorylase ein natives Protein oder eine mutierte Variante ist.

9. Verfahren nach einem der Ansprüche 1 bis 8, wobei die Saccharosephosphorylase rekombinant als Protein voller Länge oder katalytisch aktives Fragment davon oder Fusionsprotein hergestellt wird.

10. Verfahren nach einem der Ansprüche 1 bis 9, wobei die Saccharosephosphorylase in Form einer ganzen Zelle, eines zellfreien Extrakts, einer rohen, gereinigten oder immobilisierten Form verwendet wird.

11. Verfahren nach einem der Ansprüche 1 bis 10, wobei die Inkubation bei einem pH von 5,2 durchgeführt wird.

12. Verfahren nach einem der Ansprüche 1 bis 11, wobei der Inkubationsschritt für mindestens 24 Stunden, vorzugsweise für mindestens 48 Stunden, bevorzugter für mindestens 72 Stunden durchgeführt wird.

13. Verfahren nach einem der Ansprüche 1 bis 12, wobei die Inkubation in einem Temperaturbereich von etwa 30 bis 70° C, vorzugsweise von etwa 40 bis 60° C, bevorzugter von etwa 40 bis 50° C durchgeführt wird.

14. Verfahren nach einem der Ansprüche 1 bis 13, wobei die Ascorbinsäure in einem 0,3- bis 3-fachen molaren Überschuss zur Saccharose verwendet wird.

15. Verfahren nach einem der Ansprüche 1 bis 14, wobei die Menge an Saccharosephosphorylase in dem Reaktionsgemisch im Bereich von 1 U/ml bis 10.000 U/ml oder im Bereich von 5 U/ml bis 100 U/ml oder im Bereich von 10 U/ml bis 50 U/ml oder im Bereich von 20 U/ml bis 40 U/ml oder 30 U/ml ist.

16. Verfahren nach einem der Ansprüche 1 bis 15, wobei während der Inkubation zusätzliche Saccharosephosphorylase und Saccharose zur Inkubationsmischung zugesetzt wird, um die Saccharosephosphorylase im Bereich von 1 U/ml bis 10.000 U/ml oder im Bereich von 5 U/ml bis 100 U/ml oder im Bereich von 10 U/ml bis 50 U/ml oder im Bereich von 20 U/ml bis 40 U/ml oder bei 30 U/ml und Saccharose im Bereich von 100 bis 2.000 mM oder im Bereich von 250 mM bis 1.000 mM oder bei 800 mM zu halten.

17. Verfahren nach Anspruch 16, wobei Saccharosephosphorylase und Saccharose gleichzeitig oder zu unterschiedlichen Zeitpunkten zugegeben werden.

## Revendications

1. Méthode pour produire de l'acide 2-O-α-D-glucopyranosyl-L-ascorbique, comprenant les étapes successives suivantes :
a. obtention d'un mélange comprenant un donneur de glucosyle et un accepteur de glucosyle ;
b. incubation dudit mélange avec une saccharose phosphorylase ;
c. maintien du pH dans la plage de 4,8 à 5,5 durant l'incubation ;
d. éventuellement dosage de donneur de glycosyle et/ou de saccharose phosphorylase supplémentaires durant la réaction ; et
e. isolation et/ou purification d'acide 2-O-α-D-glucopyranosyl-L-ascorbique ;
dans laquelle ledit donneur de glucosyle est le 1-phosphate de glucose ou le saccharose ; et
dans laquelle ledit accepteur de glucosyle est l'acide ascorbique.

2. Méthode selon la revendication 1, dans laquelle la saccharose phosphorylase est d'origine métagénomique, microbienne, de préférence bactérienne.

3. Méthode selon la revendication 1 ou 2, dans laquelle la saccharose phosphorylase est homodimère.

4. Méthode selon l'une quelconque des revendications 1 à 3, dans laquelle la saccharose phosphorylase bactérienne est obtenue à partir d'*Agrobacterium vitis, Bifidobacterium adolescentis, Bifidobacterium longum, Escherichia coli, Escherichia coli* 06, *Lactobacillus acidophilus, Lactobacillus delbrueckii subsp. lactis, Leuconostoc mesenteroides, Listeria monocytogenes, Pseudomonas putrefaciens, Pseudomonas saccharophila, Rhodopirellula baltica, Schewanella baltica, Schewanella frigidimarina, Solibacter usitasus, Streptococcus mutans* et/ou *Synechococcus sp.*

5. Méthode selon la revendication 4, dans laquelle la saccharose phosphorylase bactérienne est obtenue à partir de *Bifidobacterium adolescentis.*

6. Méthode selon la revendication 4, dans laquelle la saccharose phosphorylase bactérienne est obtenue à partir de *Bifidobacterium longum.*

7. Méthode selon la revendication 4, dans laquelle la saccharose phosphorylase bactérienne est obtenue à partir de *Streptococcus mutans.*

8. Méthode selon l'une quelconque des revendications 1 à 7, dans laquelle la saccharose phosphorylase est une protéine native ou un variant mutant.

9. Méthode selon l'une quelconque des revendications 1 à 8, dans laquelle la saccharose phosphorylase est produite par recombinaison sous la forme d'une protéine de pleine longueur ou d'un fragment catalytiquement actif de celle-ci, ou d'une protéine de fusion.

10. Méthode selon l'une quelconque des revendications 1 à 9, dans laquelle la saccharose phosphorylase est utilisée sous la forme d'une cellule entière, d'un extrait acellulaire, ou sous une forme brute, purifiée ou immobilisée.

11. Méthode selon l'une quelconque des revendications 1 à 10, dans laquelle l'incubation est effectuée à un pH de 5,2.

12. Méthode selon l'une quelconque des revendications 1 à 11, dans laquelle l'étape d'incubation est effectuée pendant au moins 24 heures, de préférence pendant au moins 48 heures, préférentiellement pendant au moins 72 heures.

13. Méthode selon l'une quelconque des revendications 1 à 12, dans laquelle l'incubation est effectuée dans la plage de température allant d'environ 30 à 70°C, de préférence d'environ 40 à 60°C, préférentiellement d'environ 40 à 50°C.

14. Méthode selon l'une quelconque des revendications 1 à 13, dans laquelle l'acide ascorbique est utilisé en un excès molaire de 0,3 à 3 fois par rapport au saccharose.

15. Méthode selon l'une quelconque des revendications 1 à 14, dans laquelle la quantité de saccharose phosphorylase dans le mélange réactionnel est située dans la plage allant de 1 U/ml à 10 000 U/ml, ou dans la plage allant de 5 U/ml à 100 U/ml, ou dans la plage allant de 10 U/ml à 50 U/ml, ou dans la plage allant de 20 U/ml à 40 U/ml, ou est de 30 U/ml.

16. Méthode selon l'une quelconque des revendications 1 à 15, dans laquelle la saccharose phosphorylase et le saccharose supplémentaires sont ajoutés au mélange d'incubation durant l'incubation pour que la saccharose phosphorylase soit maintenue dans la plage allant de 1 U/ml à 10 000 U/ml, ou dans la plage allant de 5 U/ml à 100 U/ml, ou dans la plage allant de 10 U/ml à 50 U/ml, ou dans la plage allant de 20 U/ml à 40 U/ml, ou à 30 U/ml, et que le saccharose soit maintenu dans la plage allant de 100 à 2 000 mM, ou dans la plage allant de 250 mM à 1 000 mM, ou à 800 mM.

17. Méthode selon la revendication 16, dans laquelle la saccharose phosphorylase et le saccharose sont ajoutés simultanément ou à des points de temps différents.
